# EUROPÄISCHE PATENTSCHRIFT

(11) **EP 3 876 884 B1**
(45) Veröffentlichungstag und Bekanntmachung des Hinweises auf die Patenterteilung: **10.01.2024**
(21) Anmeldenummer: 19801816.0
(22) Anmeldetag: 07.11.2019
(51) Int. Cl.: A61F 13/00

(54) **BANDAGENAUFDRUCK**
BANDAGE IMPRINT
IMPRESSION DE BANDAGE

(30) Priorität: 09.11.2018 DE 102018128054
(43) Veröffentlichungstag der Anmeldung: 15.09.2021
(73) Patentinhaber: KOB GmbH, 67752 Wolfstein (DE)
(72) Erfinder: BIER, Martin, 67742 Herren-Sulzbach (DE)
(74) Vertreter: Paul Hartmann AG Patents & Licensing
(86) Internationale Anmeldenummer: PCT/EP2019/080464
(87) Internationale Veröffentlichungsnummer: WO 2020/094753

(56) Entgegenhaltungen:
- EP-A1- 2 175 398
- WO-A1-2015/007335
- DE-A1- 2 043 692
- DE-A1-102008 003 122
- DE-A1-102011 076 596
- DE-U1- 8 307 204
- US-A1- 2016 193 086
- US-A1- 2016 242 964

## Beschreibung

Die vorliegende Erfindung betrifft eine Bandage zum Anlegen an den menschlichen oder tierischen Körper, wobei die Bandage ein Fasern enthaltendes Material umfasst, das wenigstens an einem Längsende mit einer Schnittkantenfixierung ausgestattet ist und zusätzlich einen Bereich aufweist, der ein Informationselement enthält. Weiterhin betrifft die Erfindung ein Verfahren zur Herstellung einer derartigen Bandage.

Bandagen, die zu therapeutischen, prophylaktischen oder kosmetischen Zwecken verwendet werden wie beispielsweise Kompressionsbandagen, Stützbandagen oder Fixierbandagen, weisen üblicherweise ein Material auf, das Fasern aus natürlich oder synthetisch gewonnenen Materialien enthält. Derartige Bandagen werden in der Regel in einem ersten Arbeitsschritt als quasi-endloses Bandagenband hergestellt und anschließend auf die gewünschte Länge zugeschnitten. Dabei stellen die entstehenden Schnittkanten Bereiche dar, aus denen Fasern herausragen und sich herauslösen können. Dies kann zu Beeinträchtigungen bei der Behandlung eines menschlichen oder tierischen Körperteils oder zur Beschädigung bis zur Zerstörung der Bandage führen. Besonders tritt dieses Problem bei Bandagen auf, die mehrmals verwendet werden und zwischen den einzelnen Verwendungsvorgängen einem Reinigungsverfahren unterzogen werden. Es ist üblich, die Schnittkanten durch Abnähen, durch Aufbringen eines Leimrandes oder einer Kunststoffschicht zu fixieren.

Derartigen Bandagen ist es gemein, dass sie im Gebrauchsfall aus einer Verpackung, die technische oder anwenderbezogene Informationen in Form eines Beipackzettels oder in Kurzfassung auf der Verpackung selbst enthalten kann, entnommen werden, um dann insbesondere am menschlichen oder tierischen Körper verwendet zu werden ohne dass Beipackzettel oder Verpackung weiterhin zur Verfügung stehen.

Darüber hinaus stellt es ein Problem dar, dass auf den meisten derartigen medizinischen Produkten technische Informationen oder anwenderbezogene Informationen entweder aus Abmessungs- oder Platzgründen sowie aus anderen Gründen, die in der Benutzung der Produkte bedingt sind, nicht aufgebracht werden können.

Nichtsdestotrotz kann es zu einem späteren Zeitpunkt, insbesondere auch während oder nach der Benutzung interessant oder notwendig sein, Informationen insbesondere zu Anwendungshinweisen oder Komplikationen bei der Anwendung zu erhalten.

Ferner ist es von verschiedenen Seiten, beispielsweise von Seiten der vertreibenden Firmen, der Registrierungsbehörden oder von Seiten der Verbraucher gewünscht oder verpflichtend, eine eindeutige Kennzeichnung der Produkte vorzunehmen. Auf diese Weise soll eine sichere Identifizierung des Produkts auch dann möglich sein, wenn Verpackungsbeilagen und sonstige Begleitinformationen nicht vorliegen. Dies kann nicht immer durch Form, Größe und Aussehen des Produktes allein sichergestellt werden.

Aus der WO 03/098543 A1 ist vorbekannt, eine Methode zum fälschungssicheren Kennzeichnen von Produkten bereitzustellen, wobei hierzu vorgesehen ist, eine individuelle verschlüsselte Kennung und eine unverschlüsselte Kennung vorzusehen, wobei die verschlüsselte Kennung, wenn sie mittels des öffentlichen Schlüssels entschlüsselt ist, der unverschlüsselten Kennung entspricht. Die Kennung wird dabei mit einem geheimen Teil des Schlüssels erzeugt, so dass bei Übereinstimmung der beiden Kennungen nach dem Entschlüsseln des verschlüsselten Teils sichergestellt ist, dass der verschlüsselte Teil mit dem geheimen Teil des Schlüssels erzeugt wurde und dass daher der Hersteller des Originals den verschlüsselten Teil erzeugt hat und es sich somit um ein Originalprodukt handelt.

Aus der EP2175398 ist weiterhin bekannt, ein medizinisches Produkt, beispielsweise eine Bandage, mit einem QR-Code zu versehen.

Aus der US2016/193086 ist bekannt, Bandagen mit verschiedenen Symbolen zu versehen, beispielsweise mit Codes, die Zugang zu weiterführenden Informationen gewähren.

Die DE 20 43 692 A1 lehrt ein Verfahren zum dauerhaften Etikettieren flexibler Gegenstände, insbesondere von Textilien.

Die WO 2015/007335 A1 offenbart eine elastische Bandage mit einem langgestreckten Streifen aus einem dehnbaren, elastischen Textilmaterial, auf dem Markierungen vorgesehen sind, die als Druckindikatoren dienen können. Die Fäden oder Garne des textilen Materials können aus einem elastischen Material, das eine Mischung von thermoplastischen und elastomeren Polymeren umfasst, bestehen.

Es ist Aufgabe der Erfindung, eine Bandage bereitzustellen, welche eine Fixierung der Schnittkanten aufweist und welche auf einfache Weise einem Benutzer weitergehende Informationen zur Bandage zur Verfügung stellt, auch wenn keine weitergehenden Begleitinformationen, wie Beipackzettel mehr vorhanden sind. Gleichzeitig soll dabei sichergestellt werden, dass die bereitgestellten Informationen spezifisch das jeweilige Produkt betreffen. Schließlich soll erreicht werden, dass Schnittkantenfixierung und Zurverfügungstellen weitergehender Information in einer produktionstechnisch und ökonomisch günstigen Weise erfolgen kann.

Diese Aufgabe wird durch einen Gegenstand des Anspruchs 1 gelöst.

Eine erfindungsgemäße Bandage umfasst ein flächiges Bandmaterial mit einer Längsrichtung und einer Querrichtung sowie zwei sich in Längsrichtung gegenüberliegenden Querkanten und zwei sich in Querrichtung gegenüberliegenden Längskanten. Die Bandage kann zu einer Rolle aufgewickelt werden.

Eine erfindungsgemäße Bandage weist ein Material auf, das Fasern enthält. Dabei kann es sich sowohl um aus natürlicher Quelle erhaltene Fasern handeln als auch um auf synthetischem Weg hergestellte Fasern. Bevorzugte Materialien umfassen Fasern aus Baumwolle, Cellulose, Viskose, Cellulosederivaten, Polyethylen, Polypropylen, Polyamid, Polyester, Derivate der genannten Materialien oder Mischungen davon. Eine erfindungsgemäße Bandage kann beispielsweise ein Gewebe, ein Gewirk, ein Gestrick oder ein Vlies umfassen.

Bandagen werden in der Regel in einem ersten Schritt als Bandagenband hergestellt in quasi-endloser Form hergestellt und anschließend auf die gewünschten Maße zurechtgeschnitten. Dabei entsteht an den sich in Längsrichtung gegenüberliegenden Querkanten jeweils eine Schnittkante. Hier weist die Bandage an jeder Schnittkante einen ersten Bereich auf, der lose Fasern enthält, die aus dem bandförmigen Bandagenmaterial herausragen und sich herauslösen können. Sofern keine Fixierung dieser Schnittkanten erfolgt, können herausgelöste Fasern zu einer Beschädigung der Bandage bis hin zur Zerstörung führen. Dieser Prozess wird insbesondere durch Reinigungsprozesse wie Wasch- oder Sterilisationsvorgänge verstärkt, die bei mehrfach verwendbaren Bandagen zwischen den einzelnen Verwendungen erfolgen müssen. Darüber hinaus können herausgelöste Fasern in Kontakt mit der Haut des Lebewesens gelangen, an dem die Bandage angelegt wird. Dies kann zu Beeinträchtigungen des Tragekomforts oder zu Beschwerden wie Hautreizungen oder Kontamination der zu behandelnden Bereiche führen.

Zur Fixierung von Schnittkanten können grundsätzlich verschiedene Verfahren eingesetzt werden. Beispielsweise können die Schnittkanten vernäht oder mit einem Leimrand versehen werden, der ein Herauslösen von Fasern verhindern soll. In der vorliegenden Erfindung wird eine Schnittkantenfixierung dadurch erreicht, dass in einem als Hitzetransferdruck bekannten Verfahren eine Schicht eines thermoplastischen Materials auf die Bandage im Bereich der an der in Längsrichtung (L) liegenden Querkante aufgebracht wird und durch Einwirken von Hitze und Druck in das Material eingebracht wird.

Zur Bedruckung eines textilen Materials in einem als Hitzetransferdruck bezeichneten Verfahren wird ein auch Hitzetransfer genanntes Material, welches auf einem Träger aufgebracht ist, auf das zu bedruckende textile Material aufgelegt. Das als Hitzetransfer bezeichnete Material ist bevorzugt auf einem Träger aufgebracht, welcher mit einer Trennschicht versehen sein kann. Darauf ist eine Schicht aus einem thermoplastischen Material aufgebracht. Zwischen der Trägerschicht und der Schicht aus einem thermoplastischen Material können verschiedene weitere Schichten angeordnet sein. Ein beheizter Presstempel drückt den auf einem Träger befindlichen Hitzetransfer auf die Oberfläche des zu bedruckenden Textils, so dass die Schicht aus einem thermoplastischen Material mit der Oberfläche des zu bedruckenden textilen Materials unmittelbar in Berührung kommt. Dabei wird das thermoplastische Material auf eine Temperatur oberhalb seines Erweichungspunktes erhitzt. Durch den vom Pressstempel angelegten Druck wird das erweichte thermoplastische Material in das textile Material eingedrückt, so dass die im textilen Material enthaltenen Fasern wenigstens teilweise mit thermoplastischem Material umhüllt oder deren Zwischenräume wenigstens teilweise mit thermoplastischem Material gefüllt werden. Nach einer bestimmten Zeitspanne, die als Kontaktzeit bezeichnet wird, löst der beheizte Pressstempel seinen Druck. Schließlich wird die Trägerschicht abgelöst. Der Hitzetransfer ist nun im textilen Material bzw. auf dessen Oberfläche fixiert. Zwischen Trägerschicht und thermoplastischer Schicht können weitere Schichten angeordnet werden. Derartige Hitzetransfers können bequem hergestellt werden, beispielsweise in einem Siebdruckverfahren, bei dem nacheinander auf eine Trägerschicht eine Trennschicht, gegebenenfalls eine oder mehrere Lackschichten, gegebenenfalls eine oder mehrere Farbschichten, gegebenenfalls eine oder mehrere weitere Schichten und schließlich wenigstens eine Schicht aus einem thermoplastischen Material aufgetragen werden.

Unter einem thermoplastischen Material wird ein Material verstanden, welches in einem bestimmten Temperaturbereich plastisch verformbar ist, während es bei einer niedrigeren Temperatur keine plastischen Eigenschaften aufweist. Ferner weist ein thermoplastisches Material die Eigenschaft auf, dass der Übergang vom nicht plastischen in den plastischen Zustand durch Erwärmen reversibel ist. Das heißt bei Abkühlung auf eine Temperatur unterhalb des Temperaturbereichs, in dem das Material plastisch verformbar ist, geht das Material wieder in einen Zustand über, in dem es nicht plastisch verformbar ist. Idealerweise lässt sich der Vorgang durch wiederholtes Erwärmen und Abkühlen beliebig oft wiederholen.

Im Rahmen der vorliegenden Erfindung wird unter einem thermoplastischen Material ein Material verstanden, welches bei einer Temperatur zwischen 60°C und 300°C plastisch verformbar ist, während es bei einer Temperatur unterhalb von 60°C nicht plastisch verformbar ist. Bevorzugt handelt es sich um ein Material, welches bei einer Temperatur zwischen 80°C und 200°C plastisch verformbar ist, während es bei einer Temperatur unterhalb von 80°C nicht plastisch verformbar ist. Besonders bevorzugt handelt es sich um ein Material, welches bei einer Temperatur zwischen 100°C und 150°C plastisch verformbar ist, während es bei einer Temperatur unterhalb von 100°C nicht plastisch verformbar ist. Ein derartiges Material kann vorteilhaft bei Bandagen eingesetzt werden, welche mehrfach verwendet werden und zwischen zwei Verwendungen bei einer Temperatur von bis zu 95°C gewaschen werden. Ganz besonders bevorzugt handelt es sich um ein Material, welches bei einer Temperatur zwischen 122°C und 130°C plastisch verformbar ist, während es bei einer Temperatur von weniger als 122°C nicht plastisch verformbar ist. Ein derartiges Material kann vorteilhaft in einer Bandage eingesetzt werden, welche mehrfach verwendet und zwischen zwei Verwendungen einem Dampfsterilisationsprozess bei 121°C unterzogen wird. Geeignete thermoplastische Materialien können Polymere auf Basis von Polyolefin wie beispielsweise Polyethylen oder Polypropylen, Acrylnitril-Butadien-Styrol, Polyamid, Polylactat, Polyacrylat wie beispielsweise Polymethylmethacrylat, Polycarbonat, Polyester wie beispielsweise Polyethylenterephthalat, Polystyrol, Polyetheretherketon, Polyvinylchlorid, Polyurethan, Copolymere aus diesen Materialien oder Mischungen davon umfassen. Besonders bevorzugt wird ein Material verwendet, das eine Mischung aus einem einkomponentigen Polyesterpolyurethan auf Basis eines aromatischen Diisocyanats mit pulverisiertem Copolyamid ist, welches einen Schmelzpunkt von 128°C aufweist.

Durch Erwärmen des mehrschichtigen Materials wird die Schicht aus einem thermoplastischen Material plastisch verformbar. Durch das Anlegen von Druck kann das nun plastisch verformbare Material so auf die Bandage aufgebracht werden, dass das thermoplastische Material in das Material der Bandage eindringt. Dabei entsteht ein Bereich, in dem die Bandage eine Schicht aufweist, die Fasern enthält, die wenigstens teilweise mit einem genannten thermoplastischen Material überzogen sind oder deren Zwischenräume wenigstens teilweise mit einem thermoplastischen Material gefüllt sind. Unter einer mit einem thermoplastischen Material überzogenen Faser wird eine Faser verstanden, deren Oberfläche entlang wenigstens eines Teiles ihrer Länge über ihren gesamten Umfang oder eines Teiles davon von thermoplastischem Material bedeckt ist. Nach anschließendem Abkühlen kehrt das thermoplastische Material in einen nicht plastischen Zustand zurück. Auf diese Weise werden Fasern der Bandage durch das thermoplastische Material in der Bandage fixiert, so dass ein Herauslösen der Fasern aus dem Bandagenmaterial verhindert wird.

Wird ein derartiges Verfahren zur Enden- oder Schnittkantenfixierung verwendet, weist eine so hergestellte Bandage gegenüber Bandagen, deren Enden oder Schnittkanten in einem der gennannten Verfahren Vernähen oder Verleimen hergestellt wurde, den Vorteil auf, dass sie mit geringem maschinellem Aufwand in erheblich kürzerer Zeit hergestellt werden kann. Darüber hinaus weist eine derartige Endenfixierung eine hohe Beständigkeit gegenüber mechanischer, thermischer, sonstiger physikalischer oder chemischer Beanspruchung auf, wie sie beispielsweise in einem Waschgang oder in einem Sterilisationsverfahren auftritt. Eine derartige Endenfixierung hält bis zu 50 Waschgängen bei einer Waschtemperatur bis 95°C und einer chemischen Reinigung mit Tetrachlorethen/Perchlorethylen stand. Sie ist bis ca. 130°C überbügelbar. Die Endenfixierung verfügt über eine Licht- und Wetterbeständigkeit von bis zu 2000h und weist eine Abriebbeständigkeit gemäß DIN ISO 53754 auf.

Eine erfindungsgemäße Bandage weist einen zweiten Bereich auf, in dem die Bandage eine erste Schicht aufweist, die Fasern enthält, die wenigstens teilweise mit einem genannten thermoplastischen Material überzogen sind oder deren Zwischenräume wenigstens teilweise mit einem thermoplastischen Material imprägniert sind. Dieser zweite Bereich weist eine zweite Schicht auf, die aus einem nicht thermoplastischen Material besteht. Dieser zweite Bereich kann weitere zusätzliche Schichten aufweisen. In einer bevorzugten Ausführungsform sind der erste und der zweite Bereich miteinander verbunden.

Bei der zweiten Schicht aus einem nicht thermoplastischen Material handelt es sich um eine Lackschicht. Eine derartige Lackschicht schützt die darunter liegende Farbschicht vor mechanischer, thermischer, sonstiger physikalischer oder chemischer Beanspruchung. Insbesondere bewirkt diese Lackschicht eine Beständigkeit gegenüber Feuchtigkeit, Temperatur, Schmutz, mechanischer Einwirkung, Waschmitteln, radioaktiver Strahlung wie Alpha-, Beta- oder Gammastrahlung oder chemischen Einflüssen wie beispielsweise Beständigkeit gegenüber Ethylenoxid.

Eine erfindungsgemäße Bandage weist in besagtem zweitem Bereich zusätzlich ein Informationselement auf, welches in der zweiten Schicht angeordnet ist. Im Rahmen der vorliegenden Erfindung wird unter einem Informationselement ein Element verstanden, das so gestaltet oder ausgerüstet ist, dass es Informationen an einen Beobachter vermittelt. Diese Informationen können unmittelbar verstanden werden oder einen Zugang zu einer weiteren Informationsquelle vermitteln.

Unmittelbar verständliche Informationen sind beispielsweise Texte oder Symbole in Form von graphischen Darstellungen. Diese Texte oder Symbole können beispielsweise konkrete Angaben zum Hersteller, zur Produktidentität, dekorative Elemente, Anwendungsanleitungen oder Warnhinweise beinhalten. Sie können aber auch abstrakte Darstellungen enthalten, die erst im Zusammenwirken mit weiteren Faktoren eine Bedeutung erlangen. So kann beispielsweise ein Symbol so dargestellt sein, dass seine visuelle Erscheinung in Abhängigkeit äußerer Umstände unterschiedlich sein kann. Ein Symbol kann beispielsweise bei einer dehnbaren Bandage als Indikator für das Ausmaß der Dehnung dienen, woraus der Anwender Informationen über den Applikationszustand der Bandage erhält.

Eine Information, die einen Zugang zu einer weiteren Informationsquelle vermittelt, ist beispielsweise ein Code, der vom Anwender direkt oder mittels einer Leseeinheit verstanden oder anderweitig genutzt werden kann.

Grundsätzlich sind heute eine Vielzahl von Codes bekannt, insbesondere Barcodes, wobei derartige Codes eine optoelektronisch lesbare Schrift darstellen, die aus verschiedenen Strichen oder Elementen und Lücken besteht. Die Begriffe "Code" und "Codierung" werden dabei synonym verwendet. Diese Codes können mit optischen Lesegeräten, wie z. B. Barcodelesegeräten (Scannern) oder CCD-Kameras, aber auch Mobiltelefone und PDAs gelesen und weiterverarbeitet werden. Sie können mit konventionellen Druckverfahren wie Offset, Flexo- oder Tiefdruck, aber auch mit Laser-, Thermodirekt-, Thermotransfer- oder Tintenstrahldruck aufgebracht werden. Man unterscheidet dabei Barcodes, deren Code nur in einer Achse aufgetragen ist, also eindimensionale Codes. Daneben werden zweidimensionale Codes verwendet, in denen der Code in zwei Achsen aufgetragen wird. Diese Codes können aus gestapelten eindimensionalen Codes bestehen, die in Zeilen angeordnet sind oder in echten Flächencodes. Schließlich existieren noch sogenannte 3D-Codes, bei denen beispielsweise der Farbton, die Farbsättigung oder die Farbhelligkeit die dritte Dimension darstellt. Dabei codieren alle zweidimensionalen Codes Informationen auch senkrecht zur Hauptausrichtung.

Dabei werden die echten Flächen- oder Matrixcodes, oder auch echte Arraycodes genannt, von den gestapelten Codes unterschieden. Bei den Matrixcodes werden die Daten innerhalb einer Matrix aus Blöcken gleichmäßig codiert. Bei echten Matrixcodes spielt die Ausrichtung praktisch keine Rolle, so dass die Lesung omnidirektional möglich ist. Dies hat insbesondere bei medizinischen Produkten, wie beispielsweise Bandagen, den Vorteil, dass die Ablesung durch einen Benutzer vereinfacht ist und keine Richtungsangaben an dem Produkt angebracht werden müssen, die eine Ablesung ermöglichen. Diese omnidirektionale Ablesbarkeit ist bei allen Produkten von Vorteil, die keine vorgegebene Ausrichtung produktimmanent beinhalten.

Dabei ist es besonders bevorzugt, dass der zweidimensionale Matrixcode ein QR-Code ist. QR-Codes können sehr klein sein und haben eine nahezu unbegrenzte Haltbarkeit. Die Daten im QR-Code sind redundant vorhanden. Je nach Ausgestaltung können, selbst wenn bis zu 30% des Codes zerstört sind, die Daten noch entschlüsselt werden.

Vorteilhaft bei der Verwendung von QR-Codes ist, dass heutzutage bereits viele Mobiltelefone oder PDAs (Personal Digital Assistants) mit eingebauter Kamera über eine Software verfügen, welche das Lesen von QR-Codes ermöglicht. Für viele andere Geräte ist eine solche Software erhältlich. Erfindungsgemäß ist nun durch den Code eine Internet- oder Webadresse codiert. Da heutzutage die meisten Mobiltelefone oder PDAs neben einer Kamera einen Webbrowser enthalten, kann der Code z. B. mit dem Mobiltelefon gelesen werden und wird von der Software automatisch auf die entsprechend verschlüsselte Internetseite geleitet. Dieser Internetseite können dann unmittelbar die spezifischen für das Produkt relevanten Informationen entnommen werden, wobei ein Suchen auf der Homepage des Herstellers oder gegebenenfalls sogar allgemein im Internet, um zunächst die Herstellerdaten zu erhalten, vermieden werden kann.

Darüber hinaus können auch weitere Informationen in der Codierung enthalten sein. Diese weiteren Informationen sind dann in einem zweiten zweidimensionalen Matrixcode enthalten. Beispielsweise kann mit dem ersten zweidimensionalen Matrixcode der einheitliche Quellenanzeiger (URL) der Internetseite angegeben sein, die die anwendungsspezifische und/oder technische Daten zum medizinischen Produkt enthält, und mit einem zweiten zweidimensionalen Matrixcode eine Telefonnummer angegeben sein, die eine direkte Verbindung zu einer Beratungsstelle für das medizinische Produkt herstellt. Diese Telefonnummer kann dann unmittelbar im Mobiltelefon oder im PDA weiterverarbeitet werden und so auch gegebenenfalls eine Kontaktaufnahme mit einer Hotline oder ähnlichem ermöglichen. Dies ist vorteilhaft, um weitere Informationen z. B. hinsichtlich unerwünschten Wirkungen wie therapeutischen Komplikationen des Produktes auch dann zu erhalten, wenn aus bestimmten Gründen eine Verbindung mit der Internetseite, die in der Codierung verschlüsselt ist, nicht hergestellt werden kann oder das Lesen der Daten beispielsweise auf dem Display eines Mobiltelefons als zu unkomfortabel empfunden wird oder ein direktes Gespräch mit einer beratenden Person gewünscht ist. Gemäß einem weiterführenden Gedanken der vorliegenden Erfindung ist damit auch ein medizinisches Produkt Gegenstand der vorliegenden Erfindung, das mindestens zwei zweidimensionale Matrixcodes, insbesondere zwei QR-Codes umfasst, wobei der erste Matrixcode einen einheitlichen Quellenanzeiger einer Internetseite angibt, die anwendungsspezifische und/oder technische Daten zum medizinischen Produkt enthält und der zweite Matrixcode eine Telefonnummer, die eine direkte Verbindung zu einer Beratungsstelle für das medizinische Produkt herstellt.

Anstelle eines QR-Codes können jedoch als Codierung auch sogenannte Data-Matrix-Codes, Maxi-Codes, Aztec-Codes oder Dot-Codes/Punkt-Codes verwendet werden.

Dabei bietet der QR-Code den Vorteil, dass die Software zur Entschlüsselung des Codes heutzutage weit verbreitet ist und insbesondere frei erhalten werden kann, so dass der Zugang zu den relevanten Daten für annähernd jeden Benutzer möglich ist.

Es kann dabei vorgesehen sein, dass der Quick-Response-Code oder QR-Code, der quadratisch ist und drei Suchhilfen aufweist, die als ineinander geschachtelte helle und dunkle Quadrate in drei Ecken leicht zu erkennen sind, Symbolelemente aufweist, die als Quadrate ausgestaltet sind. Erfindungsgemäß können 21 × 21 bis einschließlich 75 × 75 Quadrate, insbesondere 21 × 21 bis einschließlich 53 x 53 Quadrate und insbesondere 21 × 21 Quadrate bis einschließlich 45 × 45 Quadrate vorgesehen sein.

Der Code kann insbesondere eine Fläche von mindestens 0,5 cm² und höchstens 20 cm² einnehmen. Hierdurch kann er angebracht werden, ohne die Verwendbarkeit und Optik der Bandage zu stören, und ist doch gleichermaßen noch gut durch den Benutzer und Anwender aufzufinden und zu erkennen. Insbesondere kann der Code zwischen 1 cm² und 15 cm² und ganz bevorzugt zwischen 1 cm² und 9 cm² groß sein.

Es kann dabei vorgesehen sein, dass eine der Fehlerkorrekturlevels Anwendung findet, die eine Rekonstruktion eines beschädigten Codes von 7% bis zu 30% zulassen. D. h., bis zu 7% oder sogar bis zu 30% des Codes können nicht mehr lesbar sein und dennoch können die Informationen dem Code entnommen werden.

In einer alternativen Ausführungsform handelt es sich bei dem Informationselement um eine Schicht, die einen Stoff aufweist, das in einem physikalischen, insbesondere einem mechanischen, thermischen oder optischen Verfahren, oder einem chemischen Verfahren verändert oder zerstört werden kann. In einem derartigen Verfahren wird das Material von einem ersten Grundzustand in einen zweiten Endzustand verändert. Dabei müssen die Eigenschaften des Materials im Grundzustand von den Eigenschaften des Materials im Endzustand durch Sinneswahrnehmung oder physikalische oder chemische Untersuchung als verschieden identifiziert werden können. Bevorzugt weist die Schicht wenigstens zwei Lagen auf, deren erste und zweite Lage unterschiedliche Farben haben, die einen derartig starken Farbkontrast zueinander aufweisen, dass das menschliche Auge sie leicht als unterschiedlich wahrnehmen kann. Die obere Farbschicht kann beispielsweise mit einem Laser so zerstört werden, dass die darunter liegende Farbschicht erkennbar wird.

In einer Weiterführung dieser Ausführungsform kann das Informationselement eine weitere Schicht aufweisen, die aus einem Stoff besteht, welcher in demselben physikalischen oder chemischen Verfahren nicht zerstört wird.

Die Zerstörbarkeit des enthaltenen Materials gewährt die Möglichkeit, dass das Informationselement nach Herstellung der erfindungsgemäßen Bandage erst mit der gewünschten Information versehen wird. Beispielsweise kann es erwünscht sein, während der industriellen Produktion erfindungsgemäßer Bandagen einzelne Bandagen mit fortlaufenden Nummern zur Codierung der Seriennummer, einer Chargennummer, Verfalldatum, Herstelldatum zu versehen. Es kann auch erwünscht sein, eine mehrfach verwendbare erfindungsgemäße Bandage während des gesamten Verwendungszeitraums mit Angaben zu versehen, die die erfolgte Nutzung, Reinigung oder Sterilisation dokumentieren.

Geeignete Verfahren zur Ausstattung des Informationselements mit der gewünschten Information nach Herstellung einer erfindungsgemäßen Bandage umfassen mechanische, chemische, thermische oder optische Verfahren. Beispielsweise kann die gewünschte Information in die erste Lage durch Kratzen, Ätzen, chemische Veränderung, Licht oder Wärme eingebracht werden. Bevorzugt weist die erste Schicht einen Farbstoff auf, der mit einem Laserpuls zerstört werden kann und dabei seine Farbe ändert.

In einer alternativen Ausführungsform handelt es sich bei dem Informationselement um ein in der Fachwelt als radio-frequency identification, kurz RFID bekanntes System. RFID kann als "Identifizierung mit Hilfe elektromagnetischer Wellen" übersetzt werden und bezeichnet eine Technologie für Sender-Empfänger-Systeme zum automatischen und berührungslosen Identifizieren und Lokalisieren von Gegenständen mit Radiowellen.

Ein RFID-System besteht aus einem Transponder (umgangssprachlich auch Funketikett genannt), der sich am oder im Gegenstand befindet und einen kennzeichnenden Code enthält, sowie einem Lesegerät zum Auslesen dieser Kennung.

RFID-Transponder können heutzutage beispielsweise über ein spezielles Druckverfahren stabiler Schaltungen aus Polymeren hergestellt werden. Derartig hergestellte RFID-Transponder besitzen eine geringe Größe, eine unauffällige Auslesemöglichkeit und einen geringen Preis.

Das Lesegerät ist in der Lage, magnetische Wechselfelder mit geringer Reichweite oder hochfrequente Radiowellen zu erzeugen, die zur Kopplung von RFID-Transponder und RFID-Lesegerät verwendet werden. Damit werden nicht nur Daten übertragen, sondern auch der Transponder mit Energie versorgt.

Das Lesegerät enthält eine den eigentlichen Leseprozess steuernde Software und eine RFID-Middleware mit Schnittstellen zu weiteren EDV-Systemen und Datenbanken.

Der Aufbau eines RFID-Transponders sieht prinzipiell eine Antenne, einen analogen Schaltkreis zum Empfangen und Senden (Transceiver) sowie einen digitalen Schaltkreis und einen permanenten Speicher vor.

RFID-Transponder verfügen über einen mindestens einmal beschreibbaren Speicher, der ihre unveränderliche Identität enthält. Werden mehrfach beschreibbare Speicher eingesetzt, können während der Lebensdauer weitere Informationen abgelegt werden, beispielsweise über die bereits erfolgte Nutzung, Reinigungs- und Sterilisationsvorgänge.

Besonders bevorzugt ist vorgesehen, dass die anwendungsspezifischen und/oder technischen Informationen die inhaltliche Wiedergabe des Packungsbeilegers, des Beipackzettels oder die Gebrauchsanweisung gemäß Medizinprodukt-Richtlinie der Bandage sind. Auf diese Weise kann sichergestellt werden, dass sämtliche in diesen Informationen enthaltenen Angaben auch nach Trennung der eigentlichen Bandage von dem Packungsbeileger bzw. dem Beipackzettel oder der Gebrauchsanweisung für den Benutzer zu jedem späteren Zeitpunkt, gegebenenfalls sogar noch nach Anwendung des Produktes, verfügbar sind.

Besonders bevorzugt ist es dabei, dass die Internetseite die Informationen oder Daten in der Landessprache wiedergibt, in der das Produkt verkauft wurde. Diese Information kann zusätzlich als weitere Information in den Code eingebracht werden, so dass der Anwender unmittelbar auf die Internetseite in der entsprechenden Landessprache weitergeleitet wird.

Als Decodiergerät kann vorzugsweise ein Mobiltelefon oder PDA eingesetzt werden, das eine Einrichtung zur Aufnahme von Bildern, insbesondere eine Kamera, sowie eine Entschlüsselungs- und Übermittlungssoftware für den Code umfasst. D. h., das Mobiltelefon oder auch, sofern als Decodiergerät ein PDA eingesetzt wird, das PDA benötigt eine Software zum Lesen derartiger, vorzugsweise QR-Codes sowie eine Software, mit der eine Verbindung ins Internet hergestellt werden kann, insbesondere einen Webbrowser.

Des Weiteren umfasst die Erfindung eine Verwendung eines echten Matrix-Codes (ArrayCodes), insbesondere eines Quick-Response-Codes (QR-Codes), zur Verschlüsselung eines einheitlichen Quellenanzeigers (engl.: Uniform Resource Locator (URL)) einer Internetseite, die anwendungsspezifische Informationen und/oder technische Daten eines medizinischen Produktes bereitstellt, auf der Außenseite eines medizinischen Produktes. Dabei kann die anwendungsspezifische Information, insbesondere die inhaltliche Wiedergabe des Packungsbeilegers, des Beipackzettels oder der Gebrauchsanweisung, gemäß Medizinprodukte-Richtlinie des medizinischen Produktes ausgebildet sein.

In einer besonderen Ausführungsform kann der Code einen Zugang zu einem Video vermitteln, welches die korrekte Anwendung der jeweiligen Bandage am menschlichen oder tierischen Körper darstellt. Bevorzugt vermittelt der Code direkt den Zugang zu einem Video in der jeweiligen Sprache des Landes oder der Region, in der die Bandage vertrieben wird. Der Zugang zu einem derartigen Video kann vermittelt werden, indem der Code zunächst den Zugang auf eine Internetseite vermittelt, von der das Video dann abgespielt werden kann. Alternativ kann der Code auch direkt den Zugang zu einem entsprechenden Video vermitteln, ohne dass der zwischenzeitige Zugang zu einer Internetseite notwendig ist.

In einer alternativen Ausführungsform handelt es sich bei einer erfindungsgemäßen Bandage um eine dehnbare, bevorzugt elastisch dehnbare Bandage. In dieser Ausführungsform weist die das Informationselement enthaltende Schicht ein Material auf, welches dehnbar, bevorzugt elastisch dehnbar ist. Hierbei stellt das Informationselement einen Dehnungsindikator dar. Das Informationselement kann in diesem Fall ein geometrisches Symbol oder ein Mikrosensorsystem darstellen. Im Falle eines geometrischen Symbols werden Symbole bevorzugt, die durch Dehnung visuell wahrnehmbar ihre Form ändern, so dass für einen Anwender das ideale Dehnungsmaß zum Anlegen der Bandage erkennbar wird. Bevorzugte Symbole sind beispielsweise Ovale, die bei idealer Dehnung Kreisform annehmen, oder Rauten bzw. Rechtecke, die bei idealer Dehnung die Form eines Quadrates annehmen.

Das Informationselement kann in einer Schicht integriert sein. Eine derartige Schicht kann aus einem Kunststoffpolymer bestehen, welches unter den bei einem Hitzetransferdruckverfahren auftretenden Bedingungen Druck und Temperatur keine plastischen Eigenschaften aufweist. Bevorzugt besteht eine derartige Schicht aus einem Polyurethan-basierten Kunststoff, besonders bevorzugt aus einem Einkomponenten-Polyesterpolyurethan auf Basis eines aromatischen Diisocyanats.

In einer bevorzugten Ausführungsform weist die Bandage in besagtem zweitem Bereich eine Farbschicht auf, die vollständig von einer Lackschicht überzogen ist. In dieser Ausführungsform ist die Farbschicht unmittelbar auf der Schicht aufgebracht, welche Fasern enthält, die wenigstens teilweise mit einem genannten thermoplastischen Material überzogen sind oder deren Zwischenräume wenigstens teilweise mit einem thermoplastischen Material imprägniert sind. Unmittelbar auf der Farbschicht befindet sich besagte Lackschicht. Eine derartige Lackschicht schützt die darunter liegende Farbschicht vor mechanischer, thermischer, sonstiger physikalischer oder chemischer Beanspruchung. Insbesondere bewirkt die Lackschicht eine Beständigkeit gegenüber Feuchtigkeit, Temperatur, Schmutz, mechanischer Einwirkung, Waschmitteln, radioaktiver Strahlung wie Alpha-, Beta- oder Gammastrahlung oder chemischen Einflüssen wie beispielsweise Beständigkeit gegenüber Ethylenoxid.

Eine derartige Lackschicht kann aus einem Kunststoffpolymer bestehen, welches bei den Bedingungen des Hitzetransferdruckverfahrens keine plastischen Eigenschaften aufweist. Bevorzugt besteht eine derartige Lackschicht aus einem Einkomponenten-Polyurethan auf Basis eines aliphatischen Diisocyanats

Eine derartige Lackschicht hält bis zu 50 Waschgängen bei einer Waschtemperatur bis 95°C und einer chemischen Reinigung mit Tetrachlorethen/Perchlorethylen stand. Sie ist bis ca. 130°C überbügelbar. Die Lackschicht sorgt für eine Licht- und Wetterbeständigkeit von bis zu 2000h und eine Abriebbeständigkeit gemäß DIN ISO 53754 auf.

Ein weiterer Gegenstand der vorliegenden Erfindung ist ein Verfahren zur Herstellung einer erfindungsgemäßen Bandage. Dabei umfasst das Verfahren die folgenden Schritte:
a) Bereitstellen eines Bandagenbandes aus einem Fasern enthaltenden Material,
b) Bereitstellen eines ersten Materials aus wenigstens zwei Lagen, dessen erste Lage aus einem thermoplastischen Material besteht und dessen zweite Lage aus einem nicht thermoplastischen Material besteht,
c) Bereitstellen eines zweiten Materials aus wenigstens zwei Lagen, dessen erste Lage aus einem thermoplastischen Material besteht und dessen zweite Lage aus einem nicht thermoplastischen Material besteht,
d) Aufbringen des genannten ersten Materials auf einen ersten Bereich,
e) Aufbringen des genannten zweiten Materials auf einen zweiten Bereich,
f) Aussetzen des ersten und des zweiten Bereiches einem Druck von mindestens 1,5bar und einer Temperatur von 60-300°C,
g) Transport des Bandagenbandes zu einer Mess-, Wickel- und Ablängenvorichtung,
h) Durchtrennen des Bandagenbandes innerhalb des ersten Bereiches von einer Längskante zur anderen Längskante.

In einem bevorzugten Verfahren wird das erste und das zweite Material auf einer Trägerbahn bereitgestellt, welche sowohl das erste als auch das zweite Material in abwechselnder Folge enthält. Die Trägerbahn besteht aus einem einseitig auf Wachsbasis beschichtetem Papier. Hierbei ist die Wachsschicht gleichmäßig auf das Papier aufgetragen und besitzt eine Dicke von 9 g/m² +/- 1 g/m². Bevorzugt besteht das Papier aus einem chlorfrei gebleichten Papierzellstoff (TCF oder ECF Methode). Alternativ besteht die Trägerbahn aus einer transparenten Polyesterfolie mit Trennschicht, wobei die Dicke der Folie 117 - 133 µm beträgt.

Besondere Bedeutung kommt dabei den Parametern in Schritt f) zu. Hier muss sichergestellt werden, dass das thermoplastische Material innerhalb der Zeitspanne, in der es Druck und Temperatur ausgesetzt wird, auf eine Temperatur erhitzt wird, bei der es plastisch verformbar ist. Diese Zeitspanne wird Kontaktzeit genannt. Gleichzeitig muss der Druck so gewählt werden, dass das thermoplastische Material in seinem plastisch verformbaren Zustand die Fasern des Bandagenmaterials bedecken und in die Faserzwischenräume eindringen kann. Je niedriger die Viskosität des plastisch verformbaren Materials ist, desto geringer ist der Druck, der notwendig ist, um das thermoplastische Material in seinem plastisch verformbaren Zustand die Fasern des Bandagenmaterials bedecken zu lassen und in die Faserzwischenräume eindringen zu lassen. Dabei ist erstrebenswert, die Kontaktzeit so kurz wie möglich zu halten, um in prozessökonomischer Weise arbeiten zu können. Bevorzugt wird dabei eine Kontaktzeit von weniger als 10s, insbesondere eine Kontaktzeit von weniger als 4s angestrebt. Um dieses Ziel zu erreichen muss eine sorgfältige Auswahl der verwendeten thermoplastischen und nicht thermoplastischen Materialien und der Prozessparameter erfolgen.

Bevorzugt wird das Verfahren in Schritt f) bei einer Temperatur von 150°C bis 250°C durchgeführt. Dies setzt voraus, dass in Schritt b) ein erstes Material bereitgestellt wird aus wenigstens zwei Lagen, dessen erste Lage aus einem bei einer Temperatur von 150-250°C plastisch verformbaren Material besteht und dessen zweite Lage aus einem bei einer Temperatur von weniger als 150°C nicht plastisch verformbaren Material besteht, und dass in Schritt c) ein zweites Material bereitgestellt wird aus wenigstens zwei Lagen, dessen erste Lage aus einem bei einer Temperatur von 150-250°C plastisch verformbaren Material besteht und dessen zweite Lage aus einem bei einer Temperatur von weniger als 150°C nicht plastisch verformbaren Material besteht.

Besonders bevorzugt wird das Verfahren in Schritt f) bei einer Temperatur von 178°C bis 220°C durchgeführt. Dies setzt voraus, dass in Schritt b) ein erstes Material bereitgestellt wird aus wenigstens zwei Lagen, dessen erste Lage aus einem bei einer Temperatur von 178-220°C plastisch verformbaren Material besteht und dessen zweite Lage aus einem bei einer Temperatur von weniger als 178°C nicht plastisch verformbaren Material besteht, und dass in Schritt c) ein zweites Material bereitgestellt wird aus wenigstens zwei Lagen, dessen erste Lage aus einem bei einer Temperatur von 178-220°C plastisch verformbaren Material besteht und dessen zweite Lage aus einem bei einer Temperatur von weniger als 178°C nicht plastisch verformbaren Material besteht.

Weiterhin bevorzugt wird das Verfahren so durchgeführt, dass die Schritte d), e) und f) eine Zeitspanne von weniger als 10s, besonders bevorzugt von 1,5s bis 4,0s umfassen.

Weiterhin bevorzugt wird das Verfahren so durchgeführt, dass in Schritt f) ein Druck von 4bar bis 6 bar angelegt wird.

Überraschender Weise wurde festgestellt, dass bei der Verwendung eines einkomponentigen Polyesterpolyurethans auf Basis eines aromatischen Diisocyanats mit pulverisiertem Copolyamid, welches einen Schmelzpunkt von 128°C aufweist, als thermoplastisches Material bei einer Temperatur von 178°C und einem Druck von 4bar bis 6bar eine Kontaktzeit von höchstens 4s, bevorzugt 1,5s bis 4s ausreichend ist.

Im Folgenden werden besondere Ausgestaltungen der vorliegenden Erfindung anhand der Figuren 1 bis 6 genauer dargestellt.
Figur 1 zeigt schematisch eine erfindungsgemäße Bandage, in der das Informationselement einen QR-Code enthält.
Figur 2 zeigt schematisch eine erfindungsgemäße Bandage, in der das Informationselement einen RFID-Transponder enthält.
Figur 3 zeigt schematisch eine erfindungsgemäße Bandage, in der das Informationselement ein geometrisches Symbol als Dehnungsindikator enthält.
Figur 4 zeigt schematisch ein Verfahren zur Herstellung einer erfindungsgemäßen Bandage.
Figur 5 zeigt einen Abschnitt eines nach einem erfindungsgemäßen Verfahren hergestellten Zwischenprodukts.
Figur 6 zeigt einen Abschnitt eines weiteren nach einem erfindungsgemäßen Verfahren hergestellten Zwischenprodukts.

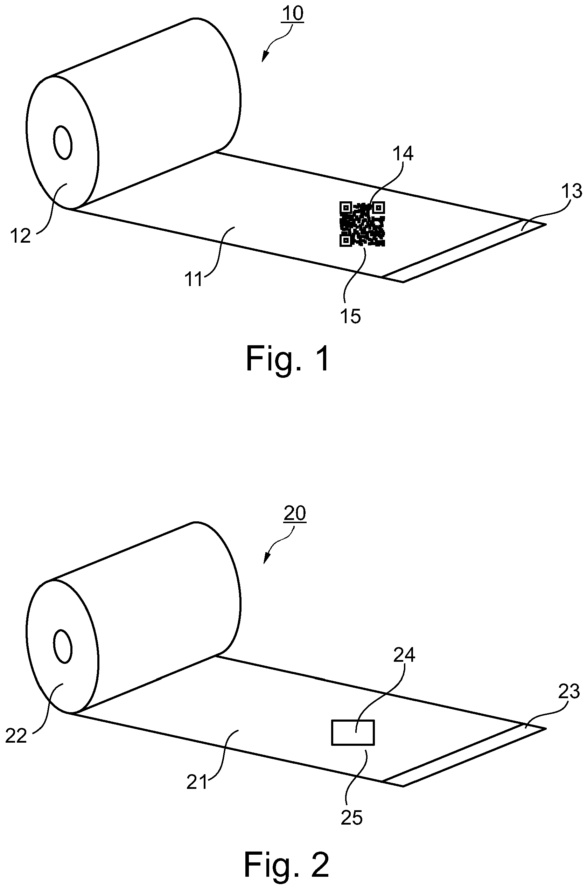

In Figur 1 ist eine erfindungsgemäße Bandage (10) gezeigt, die auf eine Rolle (12) aufgewickelt ist. Die Bandage weist ein Fasern enthaltendes Bandagenmaterial (11) auf. Am äußeren Ende der Bandage ist ein Bereich (13) vorhanden, der eine Schnittkantenfixierung aufweist. Dieser Bereich (13) enthält Fasern, welche wenigstens teilweise mit einem thermoplastischen Material überzogen oder deren Zwischenräume mit einem thermoplastischen Material imprägniert sind. Die Bandage (10) weist einen vom ersten Bereich der Schnittkante (13) verschiedenen zweiten Bereich (15) auf. Dieser weist eine erste und eine zweite Schicht auf. Die erste Schicht enthält Fasern, die wenigstens teilweise mit thermoplastischem Material überzogen oder deren Zwischenräume mit einem thermoplastischen Material imprägniert sind. Die zweite Schicht weist ein nicht thermoplastisches Material auf und enthält ein Informationselement in Form eines QR-Codes (14).

In Figur 2 ist eine erfindungsgemäße Bandage (20) gezeigt, die auf eine Rolle (22) aufgewickelt ist. Die Bandage weist ein Fasern enthaltendes Bandagenmaterial (21) auf. Am äußeren Ende der Bandage ist ein Bereich (23) vorhanden, der eine Schnittkantenfixierung aufweist. Dieser Bereich (23) enthält Fasern, welche wenigstens teilweise mit einem thermoplastischen Material überzogen oder deren Zwischenräume mit einem thermoplastischen Material imprägniert sind. Die Bandage (20) weist einen vom ersten Bereich der Schnittkante (23) verschiedenen zweiten Bereich (25) auf. Dieser weist eine erste und eine zweite Schicht auf. Die erste Schicht enthält Fasern, die wenigstens teilweise mit thermoplastischem Material überzogen oder deren Zwischenräume mit einem thermoplastischen Material imprägniert sind. Die zweite Schicht weist ein nicht thermoplastisches Material auf und enthält ein Informationselement in Form eines RFID-Transponders (24).

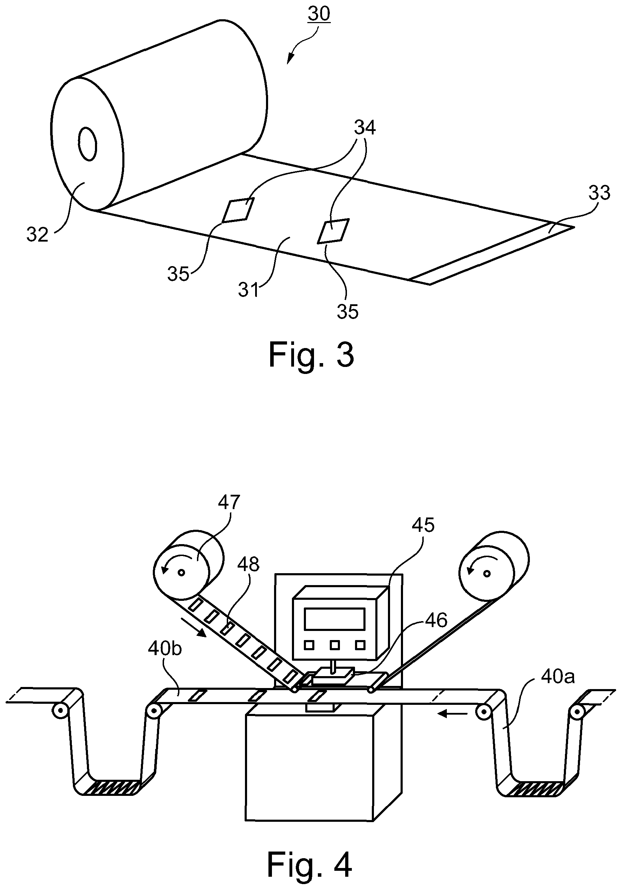

In Figur 3 ist eine erfindungsgemäße Bandage (30) gezeigt, die auf eine Rolle (32) aufgewickelt ist. Die Bandage weist ein Fasern enthaltendes Bandagenmaterial (31) auf. Am äußeren Ende der Bandage ist ein Bereich (33) vorhanden, der eine Schnittkantenfixierung aufweist. Dieser Bereich (33) enthält Fasern, welche wenigstens teilweise mit einem thermoplastischen Material überzogen oder deren Zwischenräume mit einem thermoplastischen Material imprägniert sind. Die Bandage (30) weist mehrere vom ersten Bereich der Schnittkante (33) verschiedene zweite Bereiche (35) auf. Diese weisen eine erste und eine zweite Schicht auf. Die erste Schicht enthält Fasern, die wenigstens teilweise mit thermoplastischem Material überzogen oder deren Zwischenräume mit einem thermoplastischen Material imprägniert sind. Die zweite Schicht weist ein nicht thermoplastisches Material auf und enthält ein Informationselement in Form eines geometrischen Symbols in Form einer Raute (34). Die Raute ist so gestaltet, dass sie bei Dehnung der Bandage (30) gezogen wird und bei idealer Dehnung die Form eines Quadrats annimmt.

Figur 4 zeigt schematisch ein erfindungsgemäßes Verfahren zur Herstellung einer erfindungsgemäßen Bandage. Ein Bandagenband (40a) aus einem Fasern enthaltenden Material wird zu einer Bedruckungseinheit (45) geführt. Die Bedruckungseinheit (45) weist einen beheizten Pressstempel (46) auf und ist mit einer Rolle eines Trägermaterials (47) bestückt, auf der ein als Hitzetransfer bezeichnetes mehrschichtiges Material (48) in wiederholter Abfolge aufgetragen ist. Der beheizte Pressstempel (46) drückt das Trägermaterial (47) so auf das Bandagenband (40a), dass eine Schicht des Hitzetransfers (48) aus einem thermoplastischen Material unmittelbar in Kontakt mit der Oberfläche des Bandagenbandes (40a) gerät. Durch die im Pressstempel (46) vorhandene Temperatur und den angelegten Druck wird das thermoplastische Material des Hitzetransfers (48) in einen plastisch verformbaren Zustand gebracht und in das Material des Bandagenbandes (40a) gebracht, so dass Fasern des Bandagenbandes (40a) wenigstens teilweise mit thermoplastischem Material überzogen oder deren Zwischenräume mit thermoplastsischem Material imprägniert sind. Das nun bedruckte Badagenband (40b) wird zu einer Mess-, Wickel- und Ablängenvorichtung geführt (nicht gezeigt).

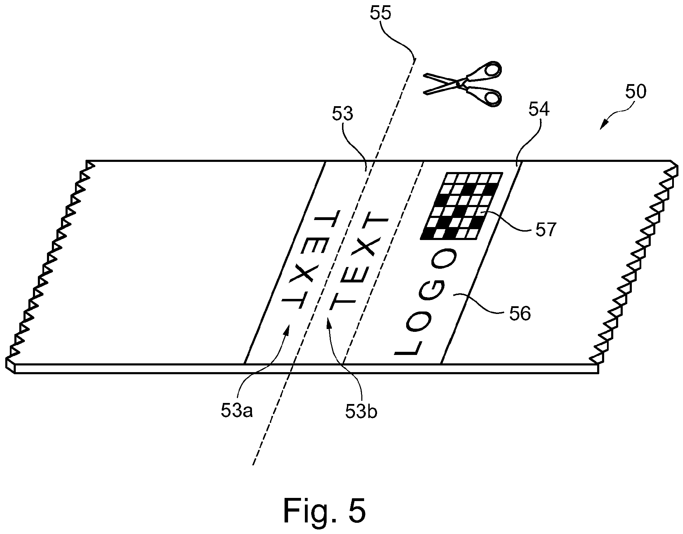

Figur 5 zeigt einen Ausschnitt einer erfindungsgemäßen Bandage (50) mit einem ersten Bereich (53) und einem zweiten Bereich (54). Der erste Bereich (53) wird in einer Ablängvorrichtung (nicht gezeigt) entlang einer Schnittlinie (55) von der einen Längsseite zur anderen Längsseite durchtrennt, so dass anschließend der erste Bereich (53) einen ersten Teilbereich (53a) umfasst, der eine Endenfixierung am Bandagenende darstellt, und einen zweiten Teilbereich (53b), der eine Endenfixierung am Bandagenanfang darstellt. Im vorliegenden Fall enthalten sowohl der erste als auch der zweite Teilbereich (53a, 53b) einen Schriftzug, der auf einer Schicht aufgebracht ist, die Fasern enthält, die wenigstens teilweise von einem thermoplastischen Material überzogen sind oder deren Zwischenräume wenigstens teilweise mit einem thermoplastischen Material imprägniert sind. Auf der Oberseite des Schriftzuges enthält der gesamte erste Bereich (53) eine Schicht aus einem Lack aus einem Einkomponenten-Polyurethan auf Basis eines aliphatischen Diisocyanats. Der Ausschnitt weist einen zweiten Bereich (54) auf, der ein Informationselement in Form einer graphischen Darstellung (56) und eines QR-Codes (57) enthält. Der zweite Bereich (54) enthält eine Schicht mit Fasern, die wenigstens teilweise von einem thermoplastischen Material überzogen sind oder deren Zwischenräume wenigstens teilweise mit einem thermoplastischen Material imprägniert sind. Unmittelbar auf dieser Schicht ist eine Farbschicht angeordnet, die sowohl eine graphische Darstellung (56) als auch einen QR-Code (57) enthält. Diese Schicht enthält Farbstoffe und ein Einkomponenten-Polyesterpolyurethan auf Basis eines aromatischen Diisocyanats. Auf der Oberseite dieser Schicht enthält der gesamte zweite Bereich (54) eine Schicht aus einem Lack aus einem Einkomponenten-Polyurethan auf Basis eines aliphatischen Diisocyanats.

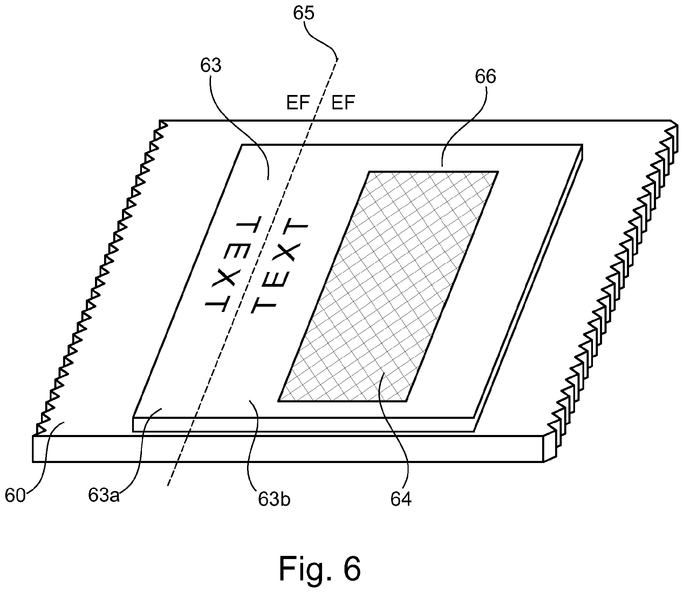

Figur 6 zeigt einen Ausschnitt einer erfindungsgemäßen Bandage (60) mit einem ersten Bereich (63) und einem zweiten Bereich (64). Der erste Bereich (63) wird in einer Ablängvorrichtung (nicht gezeigt) entlang einer Schnittlinie (65) von der einen Längsseite zur anderen Längsseite durchtrennt, so dass anschließend der erste Bereich (63) einen ersten Teilbereich (63a) umfasst, der eine Endenfixierung am Bandagenende darstellt, und einen zweiten Teilbereich (63b), der eine Endenfixierung am Bandagenanfang darstellt. Im vorliegenden Fall enthalten sowohl der erste als auch der zweite Teilbereich (63a, 63b) einen Schriftzug, der auf einer Schicht aufgebracht ist, die Fasern enthält, die wenigstens teilweise von einem thermoplastischen Material überzogen sind oder deren Zwischenräume wenigstens teilweise mit einem thermoplastischen Material imprägniert sind. Auf der Oberseite des Schriftzuges enthält der gesamte erste Bereich (63) eine Schicht aus einem Lack aus einem Einkomponenten-Polyurethan auf Basis eines aliphatischen Diisocyanats. Der Ausschnitt weist einen zweiten Bereich (66) auf, der ein Informationselement in Form einer weißen Farbschicht (64) enthält, deren weißer Farbstoff mit Hilfe eines Lasers in einem weiteren Prozessschritt eine Chargennummer angebracht werden kann (nicht gezeigt).

## Patentansprüche

1. Bandage (10, 20, 30, 50, 60) zum Anlegen an den menschlichen oder tierischen Körper aus einem Fasern enthaltenden Material umfassend ein flächiges Bandmaterial mit einer Längsrichtung und einer Querrichtung sowie zwei sich in Längsrichtung gegenüberliegenden Querkanten und zwei sich in Querrichtung gegenüberliegenden Längskanten, wobei die Bandage (10, 20, 30, 50, 60) einen ersten Bereich (13, 23, 33, 53, 63) aufweist, der an einer in Längsrichtung liegenden Querkante angrenzt, und wobei die Bandage (10, 20, 30, 50, 60) einen zweiten Bereich (15, 25, 35, 54, 66) aufweist, wobei der erste Bereich (13, 23, 33, 53, 63) eine Schicht aufweist, die Fasern enthält, die wenigstens teilweise mit einem thermoplastischen Material überzogen oder deren Zwischenräume wenigstens teilweise mit einem thermoplastischen Material imprägniert sind,
**dadurch gekennzeichnet, dass** der zweite Bereich eine erste und eine zweite Schicht aufweist, wobei die erste Schicht Fasern enthält, die wenigstens teilweise mit einem thermoplastischen Material überzogen sind oder deren Zwischenräume wenigstens teilweise mit einem thermoplastischen Material imprägniert sind, und die zweite Schicht aus einem nicht thermoplastischen Material besteht, wobei die zweite Schicht aus dem nicht thermoplastischen Material eine Lackschicht ist und die zweite Schicht ein Informationselement (14, 24, 34, 57, 64) umfasst.

2. Bandage (10, 20, 30, 50, 60) nach mindestens einem der vorgenannten Ansprüche, **dadurch gekennzeichnet, dass** es sich bei dem in der zweiten Schicht des zweiten Bereichs enthaltenen Informationselement um einen Code handelt, der einen einheitlichen Quellenanzeiger einer Internetseite angibt, die anwendungsspezifische Informationen und/oder technische Daten zur Bandage enthält.

3. Bandage (10, 50) nach Anspruch 2, **dadurch gekennzeichnet, dass** der Code ein zweidimensionaler Matrixcode (14, 57) ist.

4. Bandage (20) nach Anspruch 1, **dadurch gekennzeichnet, dass** das in der zweiten Schicht des zweiten Bereichs enthaltene Informationselement ein RFID-Transponder (24) ist.

5. Bandage (10, 20, 30, 50, 60) nach Anspruch 1, **dadurch gekennzeichnet, dass** das in der zweiten Schicht des zweiten Bereichs enthaltene Informationselement eine erste und eine zweite Lage aufweist, wobei die erste Lage die auf der äußeren Seite der Bandage angeordnete äußere Lage darstellt, und wobei die erste Lage einen Stoff aufweist, der in einem physikalischen oder chemischen Verfahren verändert oder zerstört werden kann, und wobei die zweite Lage einen Stoff aufweist, der in demselben physikalischen oder chemischen Verfahren nicht zerstört werden kann, wobei die erste und die zweite Lage verschiedene Farben aufweisen, die vom menschlichen Auge leicht als unterschiedlich wahrgenommen werden können.

6. Bandage (30) nach Anspruch 1, **dadurch gekennzeichnet, dass** die Bandage (30) dehnbar ist und die zweite Schicht des zweiten Bereichs (34) aus einem elastisch dehnbaren Material besteht und das enthaltene Informationselement ein Dehnungsindikator (34) ist.

7. Bandage (10, 20, 30, 50, 60) nach Anspruch 1, **dadurch gekennzeichnet, dass** das Fasern enthaltende Material ein Gewebe, ein Gewirke, ein Gestrick oder ein Vliesmaterial umfasst.

8. Bandage (10, 20, 30, 50, 60) nach mindestens einem der vorgenannten Ansprüche, **dadurch gekennzeichnet, dass** es sich bei den im ersten und im zweiten Bereich enthaltenen thermoplastischen Materialien um identische Materialien handelt.

9. Bandage (10, 20, 30, 50, 60) nach mindestens einem der vorgenannten Ansprüche, **dadurch gekennzeichnet, dass** es sich bei dem im ersten und im zweiten Bereich enthaltenen nicht thermoplastischen Materialien um identische Materialien handelt.

10. Bandage (10, 20, 30, 50, 60) nach mindestens einem der vorgenannten Ansprüche, **dadurch gekennzeichnet, dass** der erste Bereich eine Breite von 6 bis 20mm aufweist.

11. Bandage (10, 20, 30, 50, 60) nach mindestens einem der vorgenannten Ansprüche, **dadurch gekennzeichnet, dass** der zweite Bereich (15, 25, 35, 54, 66) mit dem ersten Bereich (13, 23, 33, 53, 63) verbunden ist.

12. Verfahren zur Herstellung einer Bandage nach einem der Ansprüche 1 bis 11 umfassend die Schritte a) Bereitstellen eines Bandagenbandes (40a) aus einem Fasern enthaltenden Material, b) Bereitstellen eines ersten Materials aus wenigstens zwei Lagen, dessen erste Lage aus einem thermoplastischen Material besteht und dessen zweite Lage aus einem nicht thermoplastischen Material besteht, c) Bereitstellen eines zweiten Materials aus wenigstens zwei Lagen, dessen erste Lage aus einem thermoplastischen Material besteht und dessen zweite Lage aus einem nicht thermoplastischen Material besteht, d) Aufbringen des genannten ersten Materials auf einen ersten Bereich, e) Aufbringen des genannten zweiten Materials auf einen zweiten Bereich, f) Aussetzen des ersten und des zweiten Bereiches einem Druck von mindestens 1,5bar und einer Temperatur von 60-300°C, g) Transport des Bandagenbandes (40a) zu einer Mess-, Wickel- und Ablängenvorichtung, h) Durchtrennen des Bandagenbandes (40a) innerhalb des ersten Bereiches von einer Längskante zur anderen Längskante.

13. Verfahren nach Anspruch 12 zur Herstellung einer Bandage nach einem der Ansprüche 1 bis 11, **dadurch gekennzeichnet, dass** Schritt f) bei einer Temperatur von 178°C bis 220°C, einem Druck von 4bar bis 6bar und einer Kontaktzeit von 1,5s bis 4s durchgeführt wird.

## Claims

1. Bandage (10, 20, 30, 50, 60) for placing on the human or animal body, composed of a fibre-containing material comprising a sheet-like band material having a longitudinal direction and a transverse direction as well as two transverse edges which are situated opposite in the longitudinal direction and two longitudinal edges which are situated opposite in the transverse direction, wherein the bandage (10, 20, 30, 50, 60) has a first region (13, 23, 33, 53, 63) which adjoins a transverse edge lying in the longitudinal direction, and wherein the bandage (10, 20, 30, 50, 60) has a second region (15, 25, 35, 54, 66), wherein the first region (13, 23, 33, 53, 63) has a layer containing fibres which are covered at least partially with a thermoplastic material or the interspaces of which are impregnated at least partially with a thermoplastic material, **characterized in that** the second region has a first and a second layer, wherein the first layer contains fibres which are covered at least partially with a thermoplastic material or the interspaces of which are impregnated at least partially with a thermoplastic material, and the second layer consists of a non-thermoplastic material, wherein the second layer composed of a non-thermoplastic material is a lacquer layer and the second layer comprises an information element (14, 24, 34, 57, 64).

2. Bandage (10, 20, 30, 50, 60) according to at least one of the preceding claims, **characterized in that** the information element incorporated in the second layer of the second region is a code which specifies a uniform resource locator of an Internet site that contains use-specific information and/or technical data relating to the bandage.

3. Bandage (10, 50) according to Claim 2, **characterized in that** the code is a two-dimensional matrix code (14, 57).

4. Bandage (20) according to Claim 1, **characterized in that** the information element incorporated in the second layer of the second region is an RFID transponder (24).

5. Bandage (10, 20, 30, 50, 60) according to Claim 1, **characterized in that** the information element incorporated in the second layer of the second region has a first and a second ply, wherein the first ply constitutes the outer ply arranged on the outer side of the bandage, and wherein the first ply comprises a substance which can be modified or destroyed in a physical or chemical process, and wherein the second ply comprises a substance which cannot be destroyed in the same physical or chemical process, wherein the first and the second ply have different colors, which can easily be perceived as different by the human eye.

6. Bandage (30) according to Claim 1, **characterized in that** the bandage (30) is stretchable and the second layer of the second region (34) consists of an elastically stretchable material and the incorporated information element is a stretch indicator (34).

7. Bandage (10, 20, 30, 50, 60) according to Claim 1, **characterized in that** the fibre-containing material comprises a woven fabric, a knitted fabric, a crocheted fabric or a nonwoven material.

8. Bandage (10, 20, 30, 50, 60) according to at least one of the preceding claims, **characterized in that** the thermoplastic materials incorporated in the first and in the second region are identical materials.

9. Bandage (10, 20, 30, 50, 60) according to at least one of the preceding claims, **characterized in that** the non-thermoplastic materials incorporated in the first and in the second region are identical materials.

10. Bandage (10, 20, 30, 50, 60) according to at least one of the preceding claims, **characterized in that** the first region has a width from 6 to 20 mm.

11. Bandage (10, 20, 30, 50, 60) according to at least one of the preceding claims, **characterized in that** the second region (15, 25, 35, 54, 66) is connected to the first region (13, 23, 33, 53, 63).

12. Method for producing a bandage according to one of Claims 1 to 11, comprising the following steps: a) providing a bandage band (40a) composed of a fibre-containing material, b) providing a first material composed of at least two plies, the first ply of which consists of a thermoplastic material and the second ply of which consists of a non-thermoplastic material, c) providing a second material composed of at least two plies, the first ply of which consists of a thermoplastic material and the second ply of which consists of a non-thermoplastic material, d) applying the mentioned first material to a first region, e) applying the mentioned second material to a second region, f) exposing the first and the second region to a pressure of at least 1.5 bar and a temperature of 60-300°C, g) transporting the bandage band (40a) to a measuring, winding and cutting-to-length device, h) severing the bandage band (40a) within the first region from one longitudinal edge to the other longitudinal edge.

13. Method according to Claim 12 for producing a bandage according to one of Claims 1 to 11, **characterized in that** step f) is carried out at a temperature from 178°C to 220°C, a pressure from 4 bar to 6 bar and a contact time from 1.5 s to 4 s.

## Revendications

1. Bandage (10, 20, 30, 50, 60) destiné à être appliqué sur le corps humain ou animal, en un matériau contenant des fibres, comprenant un matériau en bande plat ayant une direction longitudinale et une direction transversale, ainsi que deux bords transversaux opposés dans la direction longitudinale et deux bords longitudinaux opposés dans la direction transversale, le bandage (10, 20, 30, 50, 60) présentant une première zone (13, 23, 33, 53, 63) adjacente à un bord transversal situé dans la direction longitudinale, et le bandage (10, 20, 30, 50, 60) présentant une deuxième zone (15, 25, 35, 54, 66), la première zone (13, 23, 33, 53, 63) présentant une couche contenant des fibres, qui sont au moins partiellement recouvertes d'un matériau thermoplastique ou dont les interstices sont au moins partiellement imprégnés d'un matériau thermoplastique,
**caractérisé en ce que** la deuxième zone présente une première et une deuxième couche, la première couche contenant des fibres, qui sont au moins partiellement recouvertes d'un matériau thermoplastique ou dont les interstices sont au moins partiellement imprégnés d'un matériau thermoplastique, et la deuxième couche étant constituée d'un matériau non thermoplastique, la deuxième couche en matériau non thermoplastique étant une couche de vernis et la deuxième couche comprenant un élément d'information (14, 24, 34, 57, 64).

2. Bandage (10, 20, 30, 50, 60) selon au moins l'une quelconque des revendications précédentes, **caractérisé en ce que** l'élément d'information contenu dans la deuxième couche de la deuxième zone consiste en un code qui donne un localisateur de ressources uniforme d'un site Internet qui contient des informations spécifiques à l'utilisation et/ou des données techniques sur le bandage.

3. Bandage (10, 50) selon la revendication 2, **caractérisé en ce que** le code est un code matriciel bidimensionnel (14, 57).

4. Bandage (20) selon la revendication 1, **caractérisé en ce que** l'élément d'information contenu dans la deuxième couche de la deuxième zone est un transpondeur RFID (24).

5. Bandage (10, 20, 30, 50, 60) selon la revendication 1, **caractérisé en ce que** l'élément d'information contenu dans la deuxième couche de la deuxième zone présente une première et une deuxième strate, la première strate étant la strate extérieure agencée sur le côté extérieur du bandage, et la première strate présentant une substance qui peut être modifiée ou détruite par un procédé physique ou chimique, et la deuxième strate présentant une substance qui ne peut pas être détruite par le même procédé physique ou chimique, la première et la deuxième strate présentant des couleurs différentes qui peuvent être facilement perçues comme différentes par l'œil humain.

6. Bandage (30) selon la revendication 1, **caractérisé en ce que** le bandage (30) est extensible et la deuxième couche de la deuxième zone (34) est constituée d'un matériau élastiquement extensible et l'élément d'information contenu est un indicateur d'extension (34).

7. Bandage (10, 20, 30, 50, 60) selon la revendication 1, **caractérisé en ce que** le matériau contenant des fibres comprend un tissu, un tricot, un article à mailles ou un matériau non tissé.

8. Bandage (10, 20, 30, 50, 60) selon au moins l'une quelconque des revendications précédentes, **caractérisé en ce que** les matériaux thermoplastiques contenus dans la première et la deuxième zone consistent en des matériaux identiques.

9. Bandage (10, 20, 30, 50, 60) selon au moins l'une quelconque des revendications précédentes, **caractérisé en ce que** les matériaux non thermoplastiques contenus dans la première et la deuxième zone consistent en des matériaux identiques.

10. Bandage (10, 20, 30, 50, 60) selon au moins l'une quelconque des revendications précitées, **caractérisé en ce que** la première zone présente une largeur de 6 à 20 mm.

11. Bandage (10, 20, 30, 50, 60) selon au moins l'une quelconque des revendications précédentes, **caractérisé en ce que** la deuxième zone (15, 25, 35, 54, 66) est reliée à la première zone (13, 23, 33, 53, 63).

12. Procédé de fabrication d'un bandage selon l'une quelconque des revendications 1 à 11, comprenant les étapes consistant à : a) fournir une bande de bandage (40a) en un matériau contenant des fibres, b) fournir un premier matériau en au moins deux strates, dont la première strate est constituée d'un matériau thermoplastique et dont la deuxième strate est constituée d'un matériau non thermoplastique, c) fournir un deuxième matériau en au moins deux strates, dont la première strate est constituée d'un matériau thermoplastique et dont la deuxième strate est constituée d'un matériau non thermoplastique, d) appliquer ledit premier matériau sur une première zone, e) appliquer ledit deuxième matériau sur une deuxième zone, f) exposer la première et la deuxième zone à une pression d'au moins 1,5 bar et à une température de 60 à 300 °C, g) transporter la bande de bandage (40a) vers un dispositif de mesure, d'enroulement et de sectionnement, h) couper la bande de bandage (40a) à l'intérieur de la première zone d'un bord longitudinal à l'autre bord longitudinal.

13. Procédé selon la revendication 12 pour la fabrication d'un bandage selon l'une quelconque des revendications 1 à 11, **caractérisé en ce que** l'étape f) est effectuée à une température de 178 °C à 220 °C, une pression de 4 bar à 6 bar et un temps de contact de 1,5 s à 4 s.
